# EUROPÄISCHE PATENTSCHRIFT

(11) **EP 0 338 407 B1**
(45) Veröffentlichungstag und Bekanntmachung des Hinweises auf die Patenterteilung: **09.06.1993**
(21) Anmeldenummer: 89106544.3
(22) Anmeldetag: 13.04.1989
(51) Int. Cl.: C07F 9/09, C07F 9/40, C07F 9/113, C12P 13/04

(54) **Neue Alkylphosphonoserine, Verfahren zu deren Herstellung und diese enthaltende pharmazeutische Mittel**
New alkylphosphonoserines, methods for preparing them, and pharmaceutical substances containing them
Nouvelles alkylphosphonoserines, procédés pour leur préparation, et substances pharmaceutiques les contenant

(30) Priorität: 19.04.1988 DD 314868; 27.07.1988 DD 318359
(43) Veröffentlichungstag der Anmeldung: 25.10.1989
(73) Patentinhaber: Hafslund Nycomed Pharma Aktiengesellschaft, A-4021 Linz (AT)
(72) Erfinder: Brachwitz, Hans, Berlin 1071 (DD); Schönfeld, Reinhild, Berlin 1017 (DD); Langen, Peter, Berlin 1115 (DD); Paltauf, Friedrich, A-8036 Graz (AT); Hermetter, Albin, A-8036 Graz (AT)

(56) Entgegenhaltungen:
- DD-A- 238 978
- DD-A- 238 979
- DD-A- 239 405
- DE-A- 3 606 633
- DE-A- 3 639 084
- CHEMICAL ABSTRACTS, Band 87, Nr. 23, 05 Dezember 1977, Columbus, OH (US); I.L. DOERR et al., Seite 652, Nr. 184938n#
- PATENT ABSTRACTS OF JAPAN, Band 12, Nr. 372 (C-533)[3219], 05 Oktober 1988, Seite 151 C 533#

## Beschreibung

Die Erfindung betrifft neue Alkylphosphonoserine, Verfahren zu deren Herstellung und deren Verwendung.

In R. Hirt, R. Berchthold Pharmaceutica Acta Helvetica, 33(1958), 349 - 356 ist ein Herstellungsverfahren von Alkylphosphocholinen beschrieben.
Die cytostatische Wirksamkeit bzw. die Toxizität von Hexadecylphosphocholin wird in H.R. Scherf et al, Lipids 22, (1987), 927 - 929, in C. Muschol et al, Lipids 22, (1987) 930 - 934 beschrieben.
Aus DE 36 39 084 sind Phosphorsäureester, unter anderem auch Alkylphosphoserine bekannt, die als Emulgatoren oder Feuchthaltemittel verwendet werden.
Weiters sind aus DE 36 06 633 cytostatisch wirksame Phosphocholine, Phosphoserine und Phosphoethanolamine mit einem modifizierten Glycerinteil, bekannt.
Aus DD 238 978, DD 238 979 und DD 239 405 sind ferner Verfahren zur Herstellung substituierter Glycerophosphoserine bekannt, wobei diese Verbindung cytostatische Wirkung aufweisen.
Aus Chemical Abstracts, Band 87, /1977), S 652, 184938n ist ein Verfahren zur Herstellung von Phosphonat- und Ether-analogen des Phosphatidyl-L-serins bekannt.

Es konnten nun neue Alkylphosphonoserine gefunden werden, die eine ausgezeichnete cytostatische und virustatische Wirkung aufweisen.

Gegenstand der Erfindung sind Verbindungen der allgemeinen Formel I 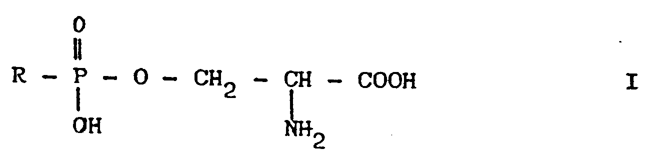
in der R einen geradkettigen oder verzweigten gesättigten oder ungesättigten aliphatischen Kohlenwasserstoffrest mit 6 - 30 C-Atomen bedeutet, der gegebenenfalls durch Halogen, die Reste OR₁, SR₁, NR₁R₂ substituiert ist, wobei R₁ und R₂ jeweils ein Wasserstoffatom oder einen Alkyl- oder Acylrest mit 1 - 6 C-Atomen bedeuten.

In der Formel I bedeutet R einen geradkettigen oder verzweigten, gesättigten oder ungesättigten aliphatischen Kohlenwasserstoffrest mit 6 - 30 C-Atomen, vorzugsweise mit 10 - 25 C-Atomen. Beispiele für solche Reste sind Decyl-, Dodecyl-, Tetradecyl-, Hexadecyl-, Octadecyl-, Arachyl-, 17-Methyl-n-octadecylreste oder der cis-9-n-Octadecenylrest.

Diese Reste können auch durch Halogen, beispielsweise durch Cl, Br, F oder durch die Reste OR₁, SR₁,NR₁R₂ substituiert sein. R₁ und R₂ bedeuten dabei unabhängig voneinander Wasserstoff oder einen Alkyl- oder Acylrest mit 1 - 6 C-Atomen, beispielsweise einen Methyl-, Ethyl-, n-Propyl-, i-Propyl, n-Butyl-, i-Butyl-, Pentyl-, Hexylrest oder beispielsweise einen Formyl-, Acetyl-, Propionyl-, Butyryl-, i-Butyrylrest.

Die Alkylphosphonoserine weisen gewisse strukturelle Beziehungen zu physiologisch aktiven natürlich vorkommenden Phospholipiden, z. B. Phosphatidylserinen auf. Sie unterscheiden sich von diesen jedoch dadurch, daß sie keine Glycerolkomponente enthalten. Im Vergleich zu den natürlich vorkommenden Phosphatidylserinen weisen die erfindungsgemäßen Alkylphosphonoserine höhere Biostabilität auf. Sie stellen potentielle Cytostatika und Virustatika dar.

Die neuen Alkylphosphonoserine können hergestellt werden, indem man
a) eine Verbindung der allgemeinen Formel II 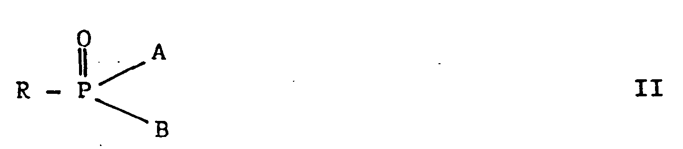 in der R die oben genannte Bedeutung hat und A und B eine Hydroxygruppe bedeuten oder gegebenenfalls die Salze dieser Verbindungen mit einem Serinderivat der allgemeinen Formel III 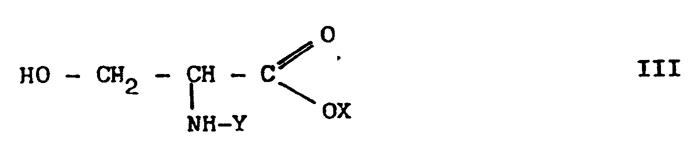 in der X Benzyl, t-Butyl, Phthalimidomethyl, Isopropyl, Benzhydryl, Y N-Benzoyloxycarbonyl, N-t-Butoxycarbonyl, N-Phthaloyl bedeutet, gegebenenfalls in Anwesenheit eines Kondensationsmittels, zu Verbindungen der allgemeinen Formel IV 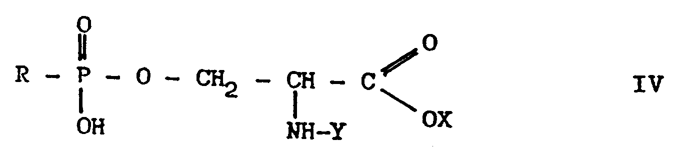 umsetzt, die Schutzgruppen abspaltet und das Produkt gegebenenfalls in ein Salz überführt oder
b) eine Verbindung der allgemeinen Formel V 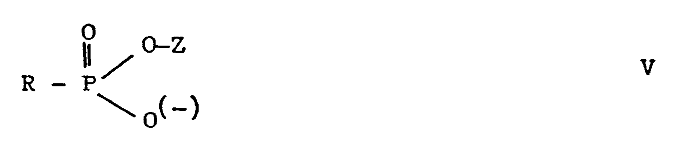 in der R die oben angegebene Bedeutung hat und Z, einen Alkylrest mit 1 - 6 C-Atomen bedeutet, der gegebenenfalls durch Cl, Br oder F substituiert sein kann oder die Reste 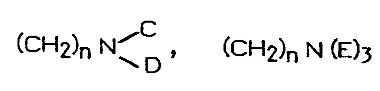 bedeuten, in denen C und D unabhängig voneinander H, oder Methyl bedeuten, E Methyl bedeutet und n 2 - 6 bedeutet, in Gegenwart von Phospholipase D mit L-Serin umsetzt.

Die als Ausgangsverbindungen verwendeten Verbindungen der allgemeinen Formel II können in an sich bekannter Weise hergestellt werden, indem man aus den entsprechenden Alkylbromid R-Br und Trimethylphosphit zunächst die Alkylphosphonsäuredimethylester synthetisiert, diese durch Umsetzung mit einem Gemisch von Trimethylsilylchlorid und Natriumjodid in die Trimethylsilylester überführt und diese durch Wassereinwirkung hydrolysiert.

Die Umsetzung der Verbindungen der allgemeinen Formel II erfolgt im allgemeinen in der Weise, daß man die Verbindungen in das Salz einer Base, beispielsweise Pyridin überführt und dieses Salz anschließend mit dem Serin-Derivat umsetzt. Die Umsetzung erfolgt vorzugsweise in Gegenwart eines Kondensationsmittels, beispielsweise in Gegenwart von 2,4,6-Triisopropylbenzolsulfochlorid unter wasserfreien Bedingungen und vorzugsweise in Gegenwart eines inerten organischen Lösungsmittels. Als Lösungsmittel kommt beispielsweise Pyridin in Frage.

Die Reaktionspartner werden üblicherweise in äquimolarer Menge eingesetzt, es ist aber auch möglich einen bis zu viermolaren Überschuß an Serinderivat einzusetzen.

Die Umsetzung erfolgt bei Temperaturen von etwa 5 - 60°C, vorzugsweise bei Raumtemperatur.
Nach Beendigung der Reaktion wird das noch vorhandene Säurechlorid auf übliche Weise, beispielsweise durch Zugabe von Wasser in der Reaktionslösung zerstört und das Produkt auf übliche Weise, beispielsweise durch Extraktion mit Diethylether, isoliert. Gegebenenfalls können die so erhaltenen Verbindungen der allgemeinen Formel IV des Formelblattes vor der Abspaltung der Schutzgruppen, beispielsweise durch Säulenchromatographie an Kieselgel weiter gereinigt werden. Es ist aber auch möglich sie ohne weitere Reinigung in die Verbindungen der allgemeinen Formel I zu überzuführen.

Dazu werden die Schutzgruppen auf übliche Weise, beispielsweise durch katalytische Hydrogenolyse, Hydrazinolyse, Behandlung mit HCl oder Ameisensäure, vorzugsweise in einem inerten Lösungsmittel abgespalten. Die Verbindungen der allgemeinen Formel I können anschließend in bekannter Weise isoliert und gegebenfalls weiter, beispielsweise chromatographisch gereinigt werden.

Die erfindungsgemäßen Alkylphosphonoserine können auch auf enzymatischem Weg hergestellt werden.
Hierbei werden Verbindungen der allgemeinen Formel V mit L-Serin in Gegenwart von Phospholipase D umgesetzt. Dazu wird eine Lösung oder Suspension der Verbindungen der allgemeinen Formel V mit einem Überschuß von L-Serin in einem wässrigen Puffersystem, beispielsweise in einer Acetat- oder Trispufferlösung bei pH-Werten von etwa 4 -8,5, vorzugsweise 5 - 6, besonders bevorzugt 5,6, in Gegenwart von etwa 0,01 -0,1 M CaCl₂ und in Gegenwart von Phospholipase D unter Zusatz von Diethylether oder einem Gemisch von Diethylether mit einem weiteren organischen Lösungsmittel, beispielsweise Chloroform umgesetzt.

Die Umsetzung erfolgt bei Temperaturen von etwa 5 - 60°C, vorzugsweise 35 - 45°C. Während der Reaktion wird das Reaktionsgemisch intensiv gerührt oder geschüttelt.
Die Reaktionsdauer beträgt etwa 0,5 - 48 Stunden. Nach beendeter Reaktion wird das Enzym Phospholipase D beispielsweise durch Zugabe einer 0,1 M EDTA-Lösung desaktiviert und die Verbindungen der allgemeinen Formel I des Formelblattes auf übliche Weise isoliert.

Die neuen Alkylphosphonoserine weisen eine starke cytostatische und virustatische Wirkung auf. Zur Bestimmung der cytostatischen Wirkung wurde die antiproliferative Wirkung bei Ehrlich-Ascites Tumorzellen untersucht. Dabei zeigte sich, daß die erfindungsgemäßen Verbindungen ausgezeichnete cytostatische Wirkung aufweisen.
Sie können daher allein oder in Mischung mit anderen Wirksubstanzen in Form üblicher galenischer Zubereitungen als Cytostatika oder Virustatika eingesetzt werden.

Die Verbindungen der allgemeinen Formel I des Formelblattes sind zur Verwendung an Menschen bestimmt und können auf übliche Weise, beispielsweise oral oder parenteral verabreicht werden. Vorzugsweise werden sie oral verabreicht, wobei die Tagesdosis etwa 0,05 bis 20 mg/kg Körpergewicht beträgt, vorzugsweise 0,05 - 5 mg/kg Körpergewicht. Der behandelnde Arzt kann jedoch abhängig vom Allgemeinzustand und dem Alter des Patienten, der entsprechenden Verbindung der allgemeinen Formel I der Art der Krankheit und der Art der Formulierung auch niedrigere oder höhere Dosen außerhalb dieses Bereiches verschreiben.

Die Verbindungen der Formel I können allein oder in Verbindung mit anderen pharmazeutisch aktiven Substanzen verabreicht werden, wobei der Gehalt der Verbindungen der allgemeinen Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, z. B. pharmazeutisch unbedenklichen Lösungsmitteln, Gelatine, Gummi arabicum, Milchzucker, Stärke, Magnesiumstearat, Talk, pflanzliche Öle, Polyalkylenglykol, Vaseline oder dgl. vor. Die pharmazeutischen Präparate können in fester Form, beispielsweise als Tabletten, Dragees, Suppositorien, Kapseln u. dgl., in halbfester Form beispielsweise als Salben oder in flüssiger Form, beispielsweise als Suspensionen oder Emulsionen vorliegen. Gegenenfalls sind sie sterilisiert und enthalten Hilfsstoffe wie Konservierungs-, Stabilisierungs-und Emulgiermittel, Salz zur Veränderung des osmotischen Drucks u. dgl. Insbesondere können pharmazeutische Präparate die erfindungsgemäßen Verbindungen in Kombination mit anderen therapeutisch wertvollen Stoff enthalten. Mit diesen können die erfindungsgemäßen Verbindungen beispielsweise zusammen mit den oben genannten Hilfs-und/oder Trägerstoffe oder Verdünnungsmitteln zu Kombinationspräparaten formuliert werden.

### Beispiel 1:

### n-Hexadecylphosphono-L-serin n-Hexadecylphosphonsäure

Eine Mischung aus n-Hexadecylbromid (4,89 g, 16 mmol) und frisch destilliertem Trimethylphosphit (2,48 g, 20 mmol) wird für 24 Stunden bei 116°C gerührt. Anschließend wird das überschüssige Trimethylphosphit im Vakuum abdestilliert und der Rückstand 24 Stunden über Phosphorpentoxid im Vakuum aufbewahrt. Der entsprechende n-Hexadecylphosphonsäuredimethylester wird aus dem Rückstand durch zweimalige Extraktion mit je 10 ml Methanol isoliert. Die beiden Methanolextrakte werden vereinigt und im Vakuum eingeengt. Der so erhaltene ölige, farblose Rückstand, der neben dem n-Hexadecylphosphonsäuremethylester noch n-Hexadecylbromid enthält, wird weiter umgesetzt in 4,7 ml wasserfreiem Acetonitril mit Trimethylsilylchlorid (1,02 g, 9,4 mmol) und Natriumjodid (1,4 g, 9,4 mmol). Die Umsetzung erfolgt unter Rühren bei Raumtemperatur und dauert 15 Minuten.
Das bei der Reaktion entstandene Natriumchlorid wird abfiltriert. Das Filtrat wird im Vakuum eingeengt und der resultierende Rückstand wird mit 4 ml Wasser versetzt, wobei durch Hydrolyse n-Hexadecylphosphonsäure entsteht. Um das gleichzeitig gebildete Me₃SiOSiMe₃ zu entfernen, wird mehrmals mit Ethanol/Wasser (1 : 1, V/V) im Vakuum nachdestilliert. Nach Behandlung des Rückstandes mit 7 ml getrocknetem Aceton werden 346 mg n-Hexadexylphosphonsäure II erhalten. DC (Kieselgel 60, Merck-Fertigplatten, Chloroform/Methanol/Wasser, 65 : 25 : 4, V/V/V): Rf = 0,15.
Elementaranalyse berechnet für C₁₆H₃₅PO₃ (306,42): C 62,71, H 11,51; gefunden C 62,81, H 11,89 (%).

### n-Hexadecylphosphono-N-t-butoxycarbonyl-L-serinbenzhydrylester

Eine Lösung von 92 mg (0,3 mmol) n-Hexadecylphosphonsäure in 1 ml Pyridin wird im Vakuum eingeengt und das so erhaltene Pyridiniumsalz anschließend über Phosphorpentoxid im Vakuum getrocknet. Danach wird es in 5 ml wasserfreiem Pyridin gelöst. 356 mg (0,96 mmol) geschütztes Serin (X = CH(C₆H₅)₂, Y = CO-O-C(CH3)3, über Phosphorpentoxid im Vakuum getrocknet, werden in 4 ml wasserfreiem Pyridin gelöst. Die beiden Lösungen werden vermischt und zum Gemisch 581 mg (1,92 mmol) Triisopropylbenzolsulfochlorid zugegeben. Das Reaktionsgemisch wird unter wasserfreien Bedingungen bei Raumtemperatur 24 Stunden gerührt. Danach wird das Gemisch im Vakuum eingeengt, mehrfach mit Toluen nachdestilliert und der Rückstand über Phosphorpentoxid im Vakuum getrocknet. Dieser wird anschließend mit 15 ml Diethylether extrahiert und dreimal mit 4 ml Diethylether nachgewaschen. Die Etherlösungen werden vereinigt und im Vakuum eingeengt. Aus dem so erhaltenen Rückstand werden durch Umkristallisieren aus Acetonitril 118 mg des n-Hexadecylphosphonoserin-Derivat IV erhalten. Eine weitere Reinigung erfolgt durch Säulenchromatographie an 8 g Kieselgel 60 (Korngröße 0,04 - 0,063 mm, 230 -400 mesh, Merck).
Das für die Chromatographie verwendete Chloroform und die Lösungsmittelgemische für die Eluierung enthalten 0,5 % wäßrigen 25%igen Ammoniak. Eluiert wird mit 50 ml Chloroform, 50 ml Chloroform/Methanol, 98 : 2, V/V, 200 ml Chloroform/Methanol, 95 : 5, V/V, 200 ml Chloroform/Methanol, 90 : 10, V/V. Die Fraktionsgröße beträgt 15 ml. Die Fraktionen 15 - 20 enthalten 72 mg reinen Hexadecylphosphono-N-t-butoxycarbonyl-L-serinbenzhydrylester.
DC (Kieselgel 60, Merck-Fertigplatten, Chloroform/Methanol, 80 : 20, V/V): Rf = 0,2.
Elementaranalyse berechnet für C₃₇H₆₁N₂PO₇ (NH₄-Salz, 676,87): C 65,65 H 9,08, N 4,14; gefunden C 65,44; H 9,15 N 3,73 (%).

### n-Hexadecylphosphono-L-serin

69 mg (0,1 mmol) der beschriebenen Verbindung werden im Vakuum über Phosphorpentoxid getrocknet und anschließend in 13 ml wasserfreiem Chloroform gelöst. Durch diese Lösung wird für 10 Minuten trockener Stickstoff und anschließend unter wasserfreien Bedingungen bei 0°C für 20 Minuten Chlorwasserstoffgas geleitet. Danach wird das Reaktionsgefäß fest verschlossen und 1 Stunde bei 0°C weitergerührt. Zur Entfernung des Chlorwasserstoffgases wird anschließend 1 Stunde lang bei Raumtemperatur Stickstoff durch da Reaktionsgemisch geleitet. Danach wird das Reaktionsgemisch im Vakuum eingeengt und der Rückstand mit einer Mischung von 10 ml Chloroform/Methanol, 2 : 1, V/V, 1 ml Wasser und 0,02 ml 25 %igem wäßrigen Ammoniak versetzt, geschüttelt und nach Phasentrennung die anfallende organische Phase abgetrennt. Die wäßrige Phase wird dreimal mit je 5 ml Chloroform/Methanol/Wasser, 2 : 1 : 0,18, V/V/V, extrahiert, die organischen Lösungen werden vereinigt und im Vakuum eingeengt. Zur Entfernung von restlichem Wasser wird mehrmals mit 1 ml Benzen/Ethanol, 2 : 3, V/V nachdestilliert. Das erhaltene Produkt, Hexadecylphosphono-L-serin I, wird über Phosphorpentoxid im Vakuum getrocknet und danach mit Aceton gewaschen.
Ausbeute: 43 mg (95 %).
Zur Entfernung von Spuren an Ammoniumchlorid wird die Substanz an 2,2 g Kieselgel 60 (Korngröße 0,04 - 0,063 mm, 230 - 400 mesh, Merck) chromatographiert. Die Säule wird mit Chloroform/Methanol/wäßrigem 25 %igen Ammoniak, 80 : 20 . 0,5, V/V/V äquilibriert. Eluiert wird mit 20 ml Chloroform/Methanol/wäßrigem 25 %igen Ammoniak, 80 : 20 : 0,5, V/V/V, 20 ml Chloroform/Methanol/wäßrigem 25%igen Ammoniak, 75 : 25 : 0,5, V/V/V, 20 ml Chloroform/Methanol/wäßrigem 25 %igen Ammoniak, 70 : 30 : 0,5, V/V/V, 20 ml Chloroform/Methanol/wäßrigem 25 %igem Ammoniak, 65 : 35 : 0,5, V/V/V, 200 ml Chloroform/Methanol/wäßrigem 25 %igen Ammoniak, 60 : 40 : 0,5, V/V/V. Die Verbindung wird aus den Fraktionen 7 - 19 durch Einengen erhalten. (Fraktionsgröße 20 ml).
DC (Kieselgel 60, Merck-Fertigplatten, Chloroform/Methanol/Aceton/Essigsäure/Wasser, 10 : 2 : 4 : 2 : 1, V/V/V/V/)V: Rf = 0,1.
Die Sichtbarmachung erfolgt mit Molybdatreagenz und mit Ninhydrinreagenz.
Elementaranalyse berechnet für C₁₉H₄₅N₂PO₆ (NH₄-Salz, Monohydrat, 428,55) : C 53,24, H 10,58, N 6,54; gefunden C 53,80, H 10,55, N 5,73 (%).
FAB-Massenspektrometrie:
Anionenspektrum: m/z 414, (M-2H+Na)⁻; m/z 392, (M-H)⁻; m/z 305 (M-CH₂CH(NH₂)COOH)⁻;
Kationsspektrum: m/z 416, (M+Na)⁺; m/z 438, (M+2Na)⁺.

### Beispiel 2:

### n-Hexadecylphosphono-L-serin

0,8 g L-Serin /7,6 mmol) werden in 1,52 ml 0,1 M Acetatpuffer (pH 5,6), der 0,09 M an CaCl₂ ist, bei 45°C gelöst. Zu dieser Lösung werden 31 mg (0,075 mmol) n-Hexadecylphosphonocholin, 1,6 ml Diethylether/Chloroform (9 : 1, V/V, ethanolfrei) und 200 mg eines Phospholipase-D-Enzympräparates, das aus Weißkohl hergestellt, eine Aktivität von 0,9 U/ml (1 Unit (U) setzt bei 27°C 1 uMol Substrat pro Minute um) im Reaktionsgemisch aufweist , zugesetzt. Das Gemisch wird 2,2 Stunden bei 45°C gerührt. Nach Abkühlen auf Raumtemperatur werden 3,31 ml 0,1 M EDTA-Lösung zugesetzt und druch Einleiten von Stickstoff die organischen Lösungsmittel entfernt. Die zurückbleibende wäßrige Phase wird mit dem 4,3fachen Volumen Chloroform/Methanol (5 : 8, V/V) 30 Minuten gerührt und vom Ungelösten (L-Serin) abgesaugt. Das Filtrat wird mit 1 Volumen Wasser und 3,7 Volumen Chloroform versetzt, die Mischung 10 Minuten geschüttelt, die organische Phase abgetrennt, im Vakuum eingeengt und der erhaltene Rückstand an Carboxymethylcellulose (CM 52 Whatman, Na⁺-Form) säulenchromatographisch getrennt. Die Elution erfolgt nacheinanden mit 75 ml Chloroform, je 300 ml Chloroform/Methanol, 9 : 1, 8 : 2, 7 : 3, anschließend 1750 ml Chloroform/Methanol, 1 : 1 (jeweils V/V). Die Fraktionsgröße beträgt 50 ml. Aus Fraktionen 20 - 39 werden 5 mg reines n-Hexadecylphosphono-L-serin (I, R = C₁₆H₃₃) erhalten.
DC (Kieselgel 60, Merck-Fertigplatte, CHCl₃/CH₃OH/ Aceton/Essigsäure/H₂O, 50 : 10 : 20 : 10 : 5, V/V/V/V//V: Rf = 0,1.

Auf analoge Weise wurden weitere Verbindungen hergestellt.

Analog Beispiel 1 wurden folgende Verbindungen hergestellt:

### Beispiel 3

### n-Decylphosphono-L-serin

Aus n-Decylphosphonsäure und geschütztem Serin (Formel III, X = CH(C₆H₅)₂, Y = CO-OC(CH₃)₃). DC (Kieselgel 60, Merck-Fertigplatten, Chloroform/Methanol/Aceton/Essigsäure/Wasser 10 : 2 : 4 : 2 : 1; V/V/V/V/V):
Rf = 0,1.

### Beispiel 4

### n-Dodecylphosphono-L-serin

Aus n-Dodecylphosphonsäure und geschütztem Serin (Formel III, X = CH(C₆H₅)₂, Y = CO-OC(CH₃)₃). DC (Kieselgel 60, Merck-Fertigplatten, Chloroform/Methanol/Aceton/Essigsäure/Wasser 10 : 2 : 4 : 2 : 1; V/V/V/V/V):
Rf = 0,1.

### Beispiel 5

### n-Tetradecylphosphono-L-serin

Aus n-Tetradedylphosphonsäure und geschütztem Serin (Formel III, X = CH(C₆H₅)₂, Y = CO-OC(CH₃)₃). DC (Kieselgel 60, Merck-Fertigplatten, Chloroform/Methanol/Aceton/Essigsäure/Wasser 10 : 2 : 4 : 2 : 1; V/V/V/V/V):
Rf = 0,1.

### Beispiel 6

### n-Octadecylphosphono-L-serin

Aus n-Octadecylphosphonsäure und geschütztem Serin (Formel III, X = CH(C₆H₅)₂, Y = CO-OC(CH₃)₃). DC (Kieselgel 60, Merck-Fertigplatten, Chloroform/Methanol/Aceton/Essigsäure/Wasser 10 : 2 : 4 : 2 : 1; V/V/V/V/V):
Rf = 0,1.

### Beispiel 7

### Arachylphosphono-L-serin

Aus Arachylphosphonsäure und geschütztem Serin (Formel III, X = CH(C₆H₅)₂, Y = CO-OC(CH₃)₃). DC (Kieselgel 60, Merck-Fertigplatten, Chloroform/Methanol/Aceton/Essigsäure/Wasser 10 : 2 : 4 : 2 : 1; V/V/V/V/V):
Rf = 0,1.

### Beispiel A

| Konzentrationsabhängige Wachstumshemmung von Ehrlich-Ascites-Tumorzellen | | | | |
|---|---|---|---|---|
| Konzentration (M) | 1 x 10⁻⁴ | 3 x 10⁻⁵ | 1 x 10⁻⁵ | 3 x 10⁻⁶ |
| Hemmung (%) | | | | |
| n-Hexadecylphospono-L-serin | 89 | 45 | 13 | 9 |

## Patentansprüche (Patentansprüche für folgende(n) Vertragsstaat(en): AT, BE, CH, DE, FR, GB, IT, LI, LU, NL, SE)

1. Verbindungen der allgemeinen Formel I 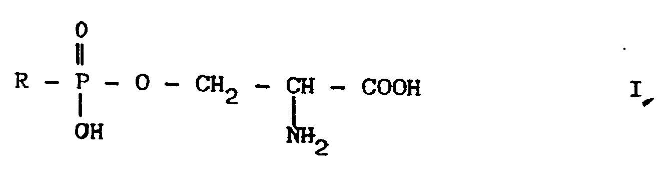 in der R einen geradkettigen oder verzweigten gesättigten oder ungesättigten aliphatischen Kohlenwasserstoffrest mit 6 - 30 C-Atomen bedeutet, der gegebenenfalls durch Halogen, die Reste OR₁, SR₁ NR₁R₂ substituiert ist, wobei R₁ und R₂ jeweils ein Wasserstoffatom oder einen Alkyl- oder Acylrest mit 1 - 6 C-Atomen bedeuten.

2. Verbindungen der allgemeinen Formel I 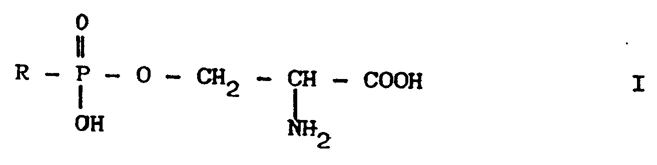 nach Anspruch 1, in der R einen geradkettigen oder verzweigten gesättigten oder ungesättigten aliphatischen Kohlenwasserstoffrest mit 10 - 25 C-Atomen, der gegebenenfalls durch Halogen, die Reste OR₁, SR₁ NR₁R₂ substituiert ist, wobei R₁ und R₂ jeweils ein Wasserstoffatom oder einen Alkyl- oder Acylrest mit 1 - 6 C-Atomen bedeuten.

3. n-Hexadecylphospono-L-serin nach einem der Ansprüche 1 oder 2.

4. Verfahren zur Herstellung von Verbindungen der allgemeinen Formel I nach Anspruch 1 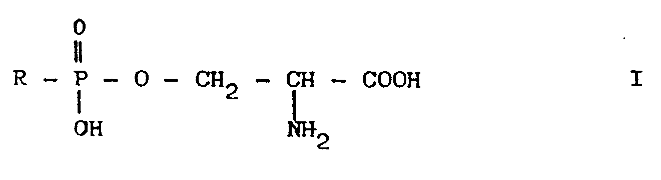 dadurch gekennzeichnet, daß man
a) eine Verbindung der allgemeinen Formel II 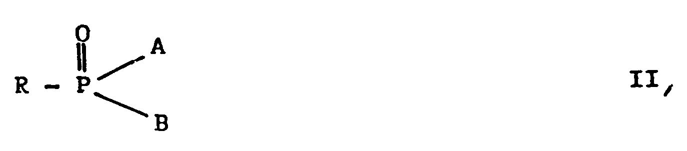 in der R die oben genannte Bedeutung hat und A und B eine Hydroxygruppe bedeuten oder gegebenenfalls die Salze dieser Verbindungen mit einem Serinderivat der allgemeinen Formel III 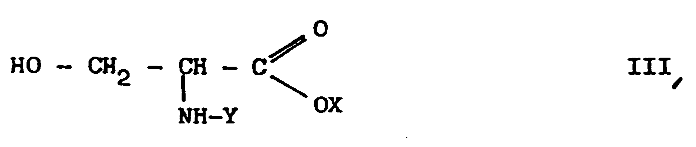 in der X Benzyl, t-Butyl, Phthalimidomethyl, Isopropyl, Benzhydryl,
Y N-Benzoyloxycarbonyl, N-t-Butoxycarbonyl N-Phthaloyl bedeutet, gegebenenfalls in Anwesenheit eines Kondensationsmittels, zu Verbindungen der allgemeinen Formel IV 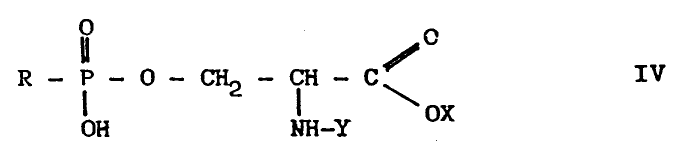 umsetzt, die Schutzgruppen abspaltet und das Produkt gegebenenfalls in ein Salz überführt oder
b) eine Verbindung der allgemeinen Formel V 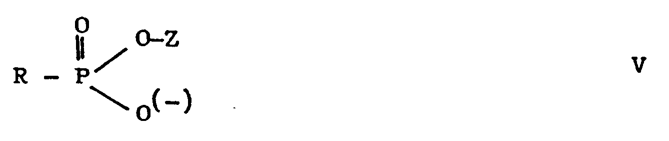 in der R die oben angegebene Bedeutung hat und Z, einen Alkylrest mit 1 - 6 C-Atomen bedeutet, der gegebenenfalls durch Cl, Br oder F substituiert ist, oder die Reste 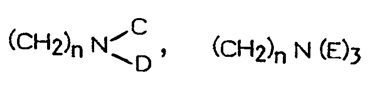 bedeuten, in denen C und D unabhängig voneinander H, oder Methyl bedeuten, E Methyl bedeutet und n 2 - 6 bedeutet, in Gegenwart von Phospholipase D mit L-Serin umsetzt.

5. Pharmazeutische Mittel, enthaltend Verbindungen der allgemeinen Formel I nach Anspruch 1 in Kombination mit üblichen galenischen Hilfs-, Träger- und/oder Verdünnungsmitteln.

6. Pharmazeutische Mittel, enthaltend Verbindungen der allgemeinen Formel I nach Anspruch 1 in Kombination mit anderen therapeutischen Wirkstoffen sowie üblichen galenischen Hilfs-Träger und/oder Verdünnungsmitteln.

## Patentansprüche (Patentansprüche für folgende(n) Vertragsstaat(en): ES, GR)

1. Verfahren zur Herstellung neuer Alkylphosphonoserine der allgemeinen Formel I 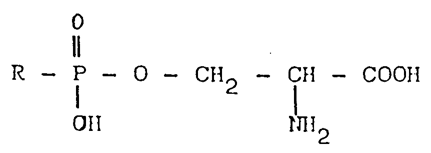 in der R einen geradkettigen oder verzweigten, gesättigten oder ungesättigten aliphatischen Kohlenwasserstoffrest mit 6 - 30 C-Atomen bedeutet, der gegebenenfalls durch Halogen, die Reste OR₁, SR₁, NR₁R₂ substituiert ist, wobei R₁ und R₂ jeweils ein Wasserstoffatom oder einen Alkyl- oder Acylrest mit 1 - 6 C-Atomen bedeuten,
dadurch gekennzeichnet, daß man
a) eine Verbindung der allgemeinen Formel II 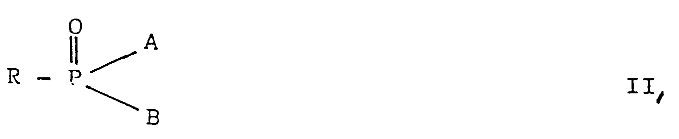 in der R wie oben definiert ist, A und B eine Hydroxygruppe bedeuten oder gegebenenfalls die Salze dieser Verbindungen mit einem Serinderivat der allgemeinen Formel 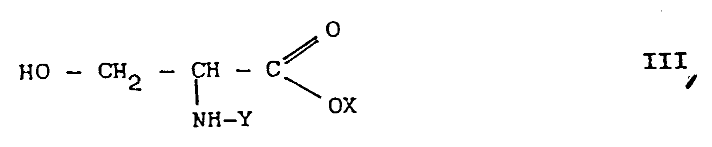 in der X Benzyl, t-Butyl, Phthalimindomethyl, Isopropyl, Benzhydryl
Y N-Benzoyloxycarbonyl, N-t-Butoxycarbonyl, N-Phthaloyl bedeutet, gegebenenfalls in Anwesenheit eines Kondensationsmittels, zu Verbindungen der allgemeinen Formel 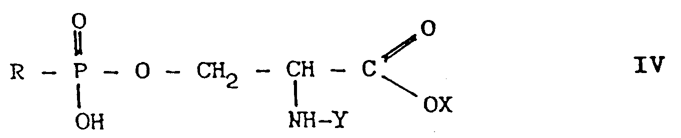 umsetzt, die Schutzgruppen abspaltet und das Produkt gegebenenfalls in ein Satz überführt oder
b) eine Verbindung der allgemeinen Formel V 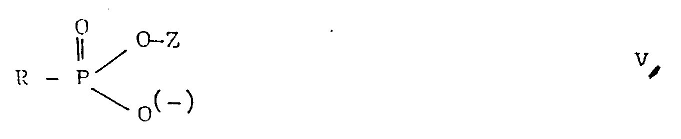 in der R die oben angegebene Bedeutung hat und Z, einen Alkylrest mit 1 - 6 C-Atomen bedeutet, der gegebenenfalls durch Cl, Br oder F substituiert ist, oder die Reste 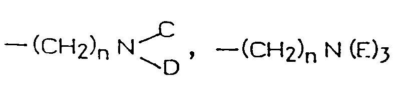 bedeuten, in denen C und D unabhängig voneinander H, oder Methyl bedeuten, E Methyl bedeutet und n 2 - 6 bedeutet, in Gegenwart von Phospholipase D mit L-Serin umsetzt.

2. Verfahren nach Anspruch 1, dadurch gekennzeichnet, daß bei der Umsetzung der Verbindungen der allgemeinen Formel II mit Verbindungen der allgemeinen Formel III als Kondensationsmittel 2,4,6-Triisopropylbenzolslfochlorid verwendet wird.

3. Verfahren nach einem der Ansprüche 1 oder 2, dadurch gekennzeichnet, daß die Verbindungen der allgemeinen Formel III in bis zu viermolerem Überschuß eingesetzt werden.

4. Verfahren nach Anspruch 1, dadurch gekennzeichnet, daß die enzymatische Umsetzung der Verbindungen der allgemeinen Formel V mit L-Serin bei einem pH von 4 - 8,5 durchgeführt wird.

5. Verfahren nach Anspruch 4, dadurch gekennzeichnet, daß die Umsetzung bei einem pH von 5 - 6 durchgeführt wird.

## Claims (Claims for the following Contracting State(s): AT, BE, CH, DE, FR, GB, IT, LI, LU, NL, SE)

1. Compounds of the general formula I 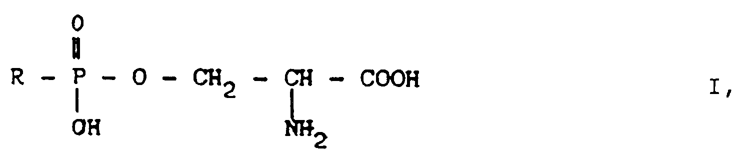 in which R denotes a straight-chain or branched, saturated or unsaturated aliphatic hydrocarbon radical having 6-30 C atoms which is optionally substituted by halogen or the radicals OR₁, SR₁ or NR₁R₂, where R₁ and R₂ in each case denote a hydrogen atom or an alkyl or acyl radical having 1-6 C atoms.

2. Compounds of the general formula I 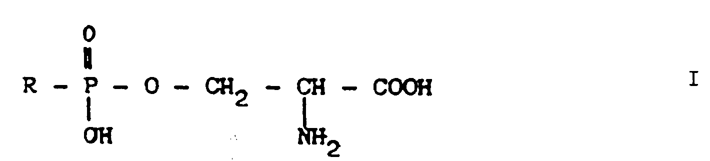 according to Claim 1, in which R denotes a straight-chain or branched, saturated or unsaturated aliphatic hydrocarbon radical having 10-25 C atoms which is optionally substituted by halogen or the radicals OR₁, SR₁ or NR₁R₂, where R₁ and R₂ in each case denote a hydrogen atom or an alkyl or acyl radical having 1-6 C atoms.

3. n-Hexadecylphosphono-L-serine according to one of Claims 1 or 2.

4. Process for the preparation of compounds of the general formula I according to Claim 1, 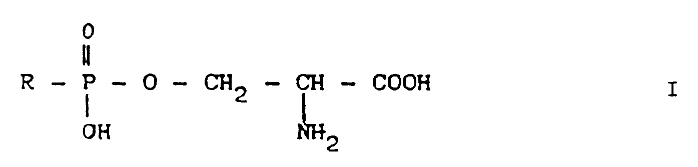 characterized in that
a) a compound of the general formula II 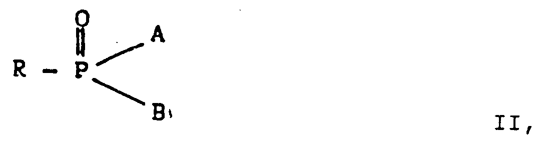 in which R has the abovementioned meaning and A and B denote a hydroxyl group or, if desired, the salts of these compounds are reacted with a serine derivative of the general formula III 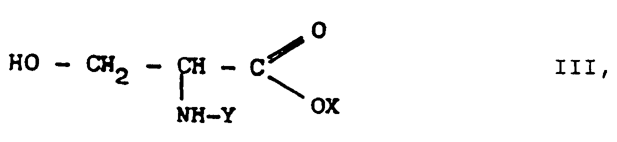 in which X denotes benzyl, t-butyl, phthalimidomethyl, isopropyl or benzhydryl,
Y denotes N-benzoyloxycarbonyl, N-t-butoxycarbonyl or N-phthaloyl, if appropriate in the presence of a condensing agent, to give compounds of the general formula IV 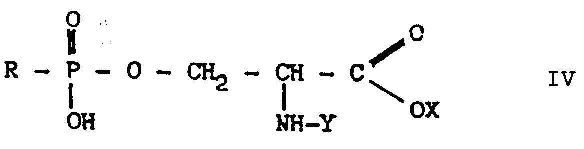 the protecting groups are removed and, if desired, the product is converted into a salt or
b) a compound of the general formula V 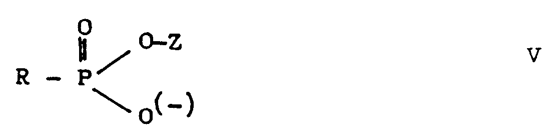 in which R has the abovementioned meaning and Z denotes an alkyl radical having 1-6 C atoms, which is optionally substituted by Cl, Br or F or denotes the radicals 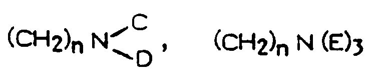 in which C and D independently of one another denote H or methyl, E denotes methyl and n denotes 2-6, is reacted with L-serine in the presence of phospholipase D.

5. Pharmaceutical agent, containing compounds of the general formula I according to Claim 1 in combination with customary pharmaceutical auxiliaries, excipients and/or diluents.

6. Pharmaceutical agent, containing compounds of the general formula I according to Claim 1 in combination with other therapeutic active compounds and customary pharmaceutical auxiliaries, excipients and/or diluents.

## Claims (Claims for the following Contracting State(s): ES, GR)

1. Process for the preparation of novel alkylphosphonoserines of the general formula I 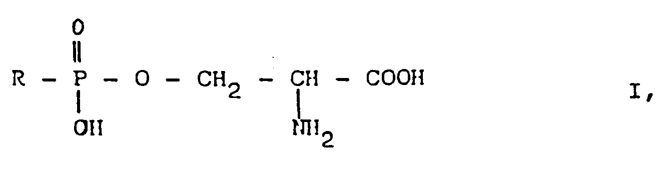 in which R denotes a straight-chain or branched, saturated or unsaturated aliphatic hydrocarbon radical having 6-30 C atoms which is optionally substituted by halogen or the radicals OR₁, SR₁ or NR₁R₂, where R₁ and R₂ in each case denote a hydrogen atom or an alkyl or acyl radical having 1-6 C atoms
characterized in that
a) a compound of the general formula II 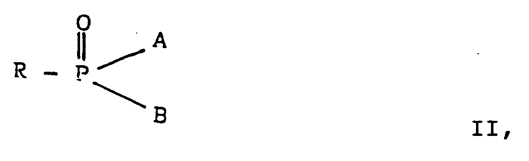 in which R is defined as above, A and B denote a hydroxyl group or, if desired, the salts of these compounds are reacted with a serine derivative of the general formula 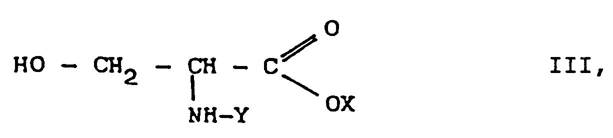 in which X denotes benzyl, t-butyl, phthalimindomethyl, isopropyl or benzhydryl
Y denotes N-benzoyloxycarbonyl, N-t-butoxycarbonyl or N-phthaloyl, if appropriate in the presence of a condensing agent, to give compounds of the general formula 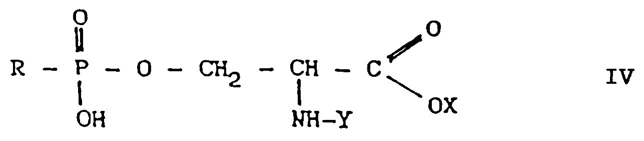 the protecting groups are removed and, if desired, the product is converted into a salt or
b) a compound of the general formula V 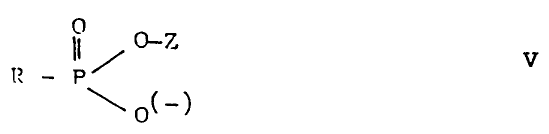 in which R has the abovementioned meaning and Z denotes an alkyl radical having 1-6 C atoms, which is optionally substituted by Cl, Br or F or denotes the radicals 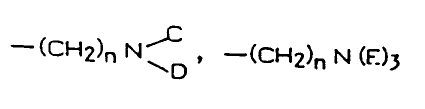 in which C and D independently of one another denote H or methyl, E denotes methyl and n denotes 2-6, is reacted with L-serine in the presence of phospholipase D.

2. Process according to Claim 1, characterised in that 2,4,6-triisopropylbenzeneslfochloride is used as condensing agent in the reaction of the compounds of the general formula II with compounds of the general formula III.

3. Process according to one of Claims 1 or 2, characterised in that the compounds of the general formula III are employed in up to four-molar excess.

4. Process according to Claim 1, characterised in that the enzymatic reaction of the compounds of the general formula V with L-serine is carried out at a pH of 4-8.5.

5. Process according to Claim 4, characterised in that the reaction is carried out at a pH of 5-6.

## Revendications (Revendications pour l'(les) Etat(s) contractant(s) suivant(s): AT, BE, CH, DE, FR, GB, IT, LI, LU, NL, SE)

1. Composés de formule générale I : 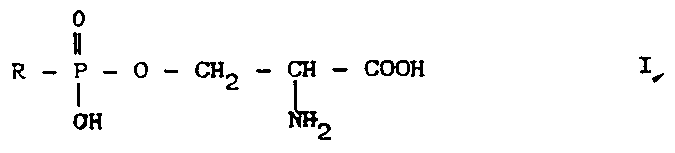 dans laquelle R désigne un reste aliphatique hydrocarboné de 6 à 30 atomes de carbone à chaîne droite ou ramifiée, saturé ou insaturé, substitué le cas échéant par un halogène, les restes OR₁, SR₁, NR₁R₂ où R₁ et R₂ désignent dans chaque cas un atome d'hydrogène ou un reste alkyle ou acyle de 1 à 6 atomes de carbone.

2. Composés de formule générale I : 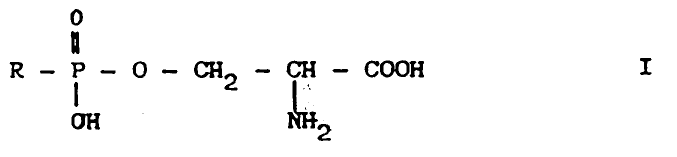 selon la revendication 1 dans laquelle R désigne un reste aliphatique hydrocarboné de 10 à 25 atomes de carbone à chaîne droite ou ramifiée, saturé ou insaturé, substitué le cas échéant par un halogène, les restes OR₁, SR₁, NR₁R₂, où R₁ et R₂ désignent dans chaque cas un atome d'hydrogène ou un reste alkyle ou acyle de 1 à 6 atomes de carbone.

3. n-hexadécylphosphono-L-sérine selon une des revendications 1 ou 2.

4. Procédé de préparation des composés de formule générale I selon la revendication 1. 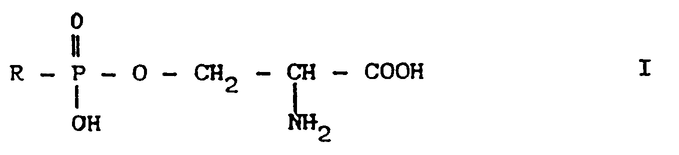 caractérisé en ce que :
a) on fait réagir un composé de formule générale II 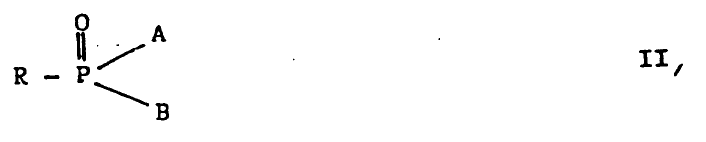 dans laquelle R a la signification indiquée ci-dessus, A et B représentent un groupe hydroxy, ou bien les sels de ces composés avec un dérivé de la sérine de formule générale III: 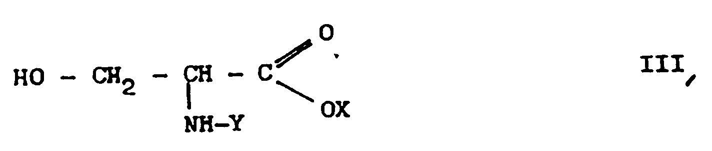 dans laquelle X signifie un groupe benzyle, tertio-butyle, phthalimidométhyle, isopropyle, benzhydrile, Y un groupe N-benzoyloxycarbonyle, N-tertio-butoxy-carbonyle, N-phthaloyle, le cas échéant en présence d'un moyen de condensation, pour obtenir un composé de formule générale IV : 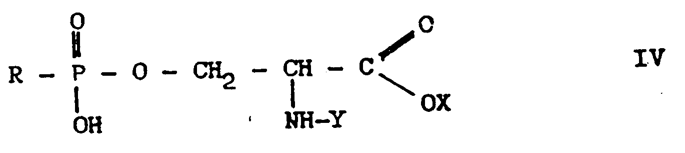 les groupes de protection étant éliminés, et le produit étant le cas échéant transformé en sel, ou bien
b) on fait réagir un composé de formule générale V 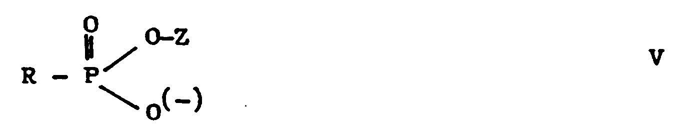 dans laquelle R a la signification sus-indiquée, Z signifie un reste alkyle de 1 à 6 atomes de carbone, substitué le cas échéant par Cl, Br ou F, ou bien les restes 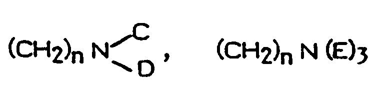 dans lesquels C et D indépendants l'un de l'autre représentent un atome d'hydrogène ou un groupe méthyle, E un groupe méthyle, n un nombre compris entre 2 et 6, en présence de phospholipase D avec la L-sérine.

5. Moyen pharmaceutique renfermant les composés de formule générale I selon la revendication 1 en combinaison avec les adjuvants, les composés vecteurs et/ou les diluants galéniques usuels.

6. Moyen pharmaceutique renfermant les composés de formule générale I en combinaison avec d'autres principes actifs thérapeutiques ainsi que les adjuvants, les composés vecteurs et/ou les diluants galéniques habituels.

## Revendications (Revendications pour l'(les) Etat(s) contractant(s) suivant(s): ES, GR)

1. Procédé de préparation de nouvelles alkylphosphonosérines de formule générale I: 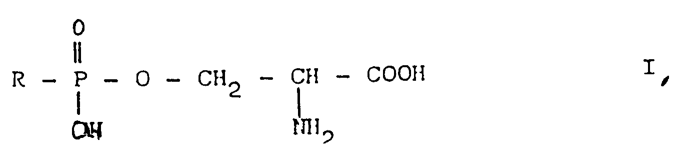 dans laquelle R représente un reste hydrocarboné aliphatique à chaîne droite ou ramifiée, saturé ou insaturé, de 6 à 30 atomes de carbone, substitué le cas échéant par des halogènes les restes OR₁, SR₁, NR₁R₂ où R₁ et R₂ désignent dans chaque cas un atome d'hydrogène ou un reste alkyle ou acyle de 1 à 6 atomes de carbone, caractérisé en ce qu'on fait réagir un composé de formule générale II 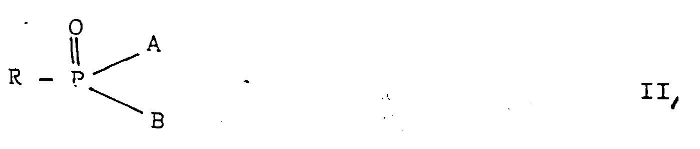 dans laquelle A et B signifient un groupe hydroxy et R est défini comme ci-dessus, ou le cas échéant les sels de ces composés, avec un dérivé de la sérine de formule générale III 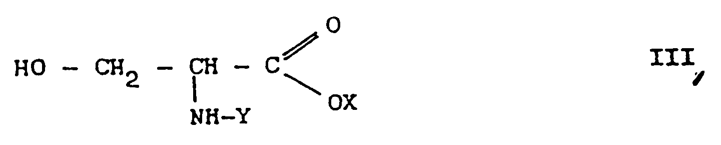 dans laquelle X signifie un groupe benzyle, tertio-butyle, phtalimidométhyle, isopropyle, benzhydrile, Y un groupe N- benzoyloxycarbonyle, N-tertiobutoxycarbonyle, n-phthaloyle, le cas échéant en présence d'un moyen de condensation, en composés de formule générale IV 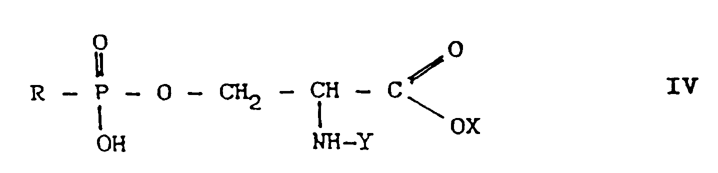 les groupes de protection étant éliminés et le produit étant transformé le cas échéant en sel, ou bien
b) on fait réagir un composé de formule générale V 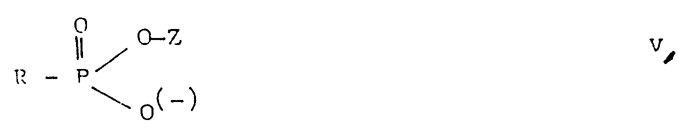 dans laquelle R a la signification indiquée ci-dessus et Z désigne un reste alkyle de 1 à 6 atomes de carbone, substitué le cas échéant par Cl, Br ou F, ou bien les restes 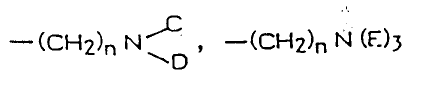 dans lesquels C et D indépendants l'un de l'autre signifient H ou un groupe méthyle, E signifie un groupe méthyle, et n un nombre compris entre 2 et 6 en présence de phospholipase D avec la L-sérine.

2. Procédé selon la revendication 1, caractérisé en ce que au cours de la réaction des composés de formule générale II avec les composés de formule générale III on utilise comme moyen de condensation le sulfochlorure 2,4,6 triisopropylbenzène.

3. Procédé selon l'une des revendications 1 ou 2, caractérisé en ce que les composés de formule générale III sont utilisés en excès jusqu'à 4 fois molaire.

4. Procédé selon la revendication 1, caractérisé en ce que la réaction enzymatique des composés de formule générale V avec la L-sérine est conduite à un pH de 4 à 8,5

5. Procédé selon la revendication 4, caractérisé en ce que la réaction est conduite à un pH de 5 à 6.
